# EUROPEAN PATENT SPECIFICATION

(11) **EP 4 414 358 B1**
(45) Date of publication and mention of the grant of the patent: **12.11.2025**
(21) Application number: 23382130.5
(22) Date of filing: 13.02.2023
(51) Int. Cl.: C07C 69/14, C07C 69/24, A01P 17/00, A01N 1/00

(54) **COMPOUNDS AND COMPOSITIONS THAT ATTRACT THE SPECIES PSEUDOCOCCUS LONGISPINUS, ATTRACTANT DEVICE AND METHODS FOR CONTROLLING AND/OR MONITORING THE PEST**
VERBINDUNGEN UND ZUSAMMENSETZUNGEN, DIE DIE SPEZIES PSEUDOCOCCUS LONGIPINUS ANZIEHEN, LOCKMITTELVORRICHTUNG UND VERFAHREN ZUR BEKÄMPFUNG UND/ODER ÜBERWACHUNG DER SCHÄDLINGE
COMPOSÉS ET COMPOSITIONS ATTIRANT LES ESPÈCES PSEUDOOCOCCUS LONGISPINUS, DISPOSITIF ATTRACTIF ET PROCÉDÉS DE LUTTE ET/OU DE SURVEILLANCE DES NUISIBLES

(43) Date of publication of application: 14.08.2024
(73) Proprietor: Universitat Politècnica de València, 46022 Valencia (ES); Ecologia Y Proteccion Agricola, S.L., 46240 Carlet (ES); Universitat De València, Estudi General, 46010 València (ES)
(72) Inventor: NAVARRO FUERTES, Ismael, 46010 Valencia (ES); VACAS GONZALEZ, Sandra, 46022 Valencia (ES); MARZO BARGUES, Javier, 46240 Carlet - Valencia (ES); NAVARRO LLOPIS, Vicente, 46022 Valencia (ES); LUNA ATANCE, Raul, 46240 Carlet - Valencia (ES); CARBONELL GARCIA, Alejandro, 46240 Carlet - Valencia (ES); PRIMO MILLO, Jaime, 46022 Valencia (ES)
(74) Representative: ABG Intellectual Property Law, S.L.

(56) References cited:
- WO-A1-2021/064719
- JOCELYN G. MILLAR ET AL: "Sex Pheromone of the Longtailed Mealybug: A New Class of Monoterpene Structure", ORGANIC LETTERS, vol. 11, no. 12, 18 June 2009 (2009-06-18), pages 2683 - 2685, XP055108751, ISSN: 1523-7060, DOI: 10.1021/ol802164v

## Description

### Technical field

The present invention falls within the technical field of agricultural pest control, in particular it refers to compounds and compositions that attract insects of the species *Pseudococcus longispinus.*

### Background

*Pseudococcus longispinus* (Targioni-Tozzetti; Hemiptera: Coccoidea: Pseudococcidae), also known as long-tailed mealybug, is a polyphagous and cosmopolitan pest that attacks a wide variety of plants, including fruits, vegetables and ornamental plants, causing serious damage to leaves, bark, branches, fruits and roots (Gillani, W., Copland, M. & Raja, S. (2011) Effect of different temperatures and host plants on the biology of the long-tailed mealybug Pseudococcus longispinus (Targioni and Tozzetti) (Homoptera: Pseudococcidae). Biological Sciences-PJSIR, 54(3), 142-151). This mealybug takes its name from the posterior pair of waxy filaments arising from the last abdominal segment in adult females, which are as long as or longer than the rest of the body. It has been described on 209 genera of 98 families of plants (Garcia-Morales M., Denno, B.D., Miller, D.R., Miller, G.L., Ben-Dov, Y. & Hardy, N.B. (2016) ScaleNet: A literature-based model of scale insect biology and systematics. Database) but, prominently, it is known as a very damaging pest for numerous crops of economic importance, among which are apple, pear, persimmon, grape, citrus, avocado, banana and other tropical fruits (Mani, M. & Shivaraju, C. (2016) Management of Mealybugs in Agricultural and Horticultural Crops, in Mani, M. and Shivaraju, C. (Eds.) Mealybugs and their management in agricultural and horticultural crops. Springer India, New Delhi, India, pp. 239-653). Like the rest of the pseudococcids, it feeds on sap and produces honeydew that causes the proliferation of saprophytic fungi, as well as a decrease in the photosynthetic rate, loss of plant vigor, and premature ripening and fruit drop. In addition, due to its feeding habits, *P. longispinus* is capable of acquiring and transmitting viruses, being known the effective transmission of some of the families Betaflexiviridae (GVA, La Notte, P., Buzkan, N., Choueiri, E., Minafra, A. & Martelli, G. P. (1997) Acquisition and transmission of grapevine virus A by the mealybug Pseudococcus longispinus. Journal of Plant Pathology, 79-85) and Closteroviridae (GLRaV-3, Petersen, C. L. & Charles, J. G. (1997) Transmission of grapevine leafroll-associated closteroviruses by Pseudococcus longispinus and P. calceolariae. Plant Patholology, 46(4), 509-515), among others.

Although their cryptic habits and the existence of overlapping generations make it difficult to manage these insects, the control of *P. longispinus* is currently based on the application of conventional chemical treatments, with only a few active materials currently available such as mineral oil and spirotetramat (IVIA 2022. Gestión integrada de Plagas y Enfermedades en Caqui).

The ban by the European Commission on the use of the material methyl-chlorpyrifos in January 2020 has caused great concern among farmers since the few available materials that are still recommended are of very limited efficacy. On the other hand, the time of application of these control measures is critical to achieve effective control. Although this time is not precisely defined, it must correspond to the maximum of sensitive forms of the pest, which is not easy to determine in species that are not very synchronized. Regarding biological control tools, research is being carried out in this regard and evaluating the efficacy of some Encyrtidae parasitoids such as *Acerophagus angustifrons* (Gahan), *Anagyrus fusciventris* (Girault), *Cryptanusia comperei* (Timberlake) and *Leptomastix algirica Trjapitzin.* Regarding the predators, the coccinellid *Cryptolaemus montrouzieri* Mulsant is used in biological control programs against pseudococcids, being introduced for the first time in Spain to control populations of *Planococcus citri* Risso in citrus plants. *Cryptolaemus montrouzeri* has been observed feeding on *P. longispinus* in the field, exerting control during the summer, but it is not capable of controlling populations on its own at the end of the season (September-December) in the case of persimmon cultivation in Spain, or other crops such us avocado, citrus, grapes, pear, persimmon, and pineapple (Faber B. A et al. 2007, UC IPM pest management guidelines: Avocado. UC ANR Publication 3436. University of California Agriculture and Natural Resources; Furness G. O. 1976, Australian Journal of Zoology 24: 237-247; Dentener P. R. et al. 1997, Postharvest Biology and Technology 12: 255-264), and ornamental plants, including species of cycads (Culbert 2010. Florida Coonties and Atala Butterflies: ENH117/MG347, rev. 3/2010. EDIS, 2010(2)) and orchids (Kot et al. 2015, Bulletin of Entomological Research 105: 373-380; Ray and Hoy 2014, Florida Entomologist 97: 972-978) in Australia, America, Asia and European countries.

In this context, the lack of effective methods for controlling *P. longispinus* is evident and farmers and producer associations need both direct control measures and tools for their detection and monitoring of populations. The application of the detection and monitoring of pseudococcid populations is of vital importance to improve the control of the pest in question, both in agricultural and ornamental ecosystems. However, it usually consists of a laborious visual inspection of the plant material in search of the stages of the insect. Sticky traps baited with sex pheromone are a great tool for monitoring the flight of males in a more comfortable and sensitive way than visual inspection (Navarro-Llopis, V., Primo, J., Navarro, I., & Vacas, S (2019) Seguimiento y distribución del cotonet de Sudáfrica Delotococcus aberiae Delotto (Hemiptera: Pseudococcidae) en la Comunidad Valenciana mediante trampas cebadas con su feromona sexual. Phytoma España, 311, 56-61). Many of the economically important pseudococcid species reproduce sexually, with females producing a sex pheromone to attract conspecific males. So far, the chemical structure of sex pheromones of 32 species of coccoid insects belonging to the families Diaspididae, Matsucoccidae, Margarodidae and Pseudococcidae is known (Franco, J. C., Cocco, A., Lucchi, A., Mendel, Z., Suma, P., Vacas, S., Mansour, R. & Navarro-Llopis, V. (2021) Scientific and technological developments in mating disruption of scale insects. Entomologia Generalis 42, 251-273) and some of them are used for the detection and monitoring of their populations. On the other hand, the use of these sex pheromones in commercial treatments for the direct control of coccoid pests (*Aonidiella aurantii* Maskell; Scalebur^{®}, Ecologia y Protección Agricola, Valencia) and *Planococcus ficus Signoret* (CheckMate^{®} VMB-XL, Suterra, Bend, USA) is known and characterized by the use of the mating disruption technique (where the male is unable to find the female through the action of various mechanisms causing the interruption of copulation) or the attraction and death of males (*Delottococcus aberiae* De Lotto; *A. aurantii, P. citri,* Vynyty^{®}, Bayer Cropscience).

The sex pheromone of the species *P. longispinus* was described by Millar et al. (Sex pheromone of the longtailed mealybug: a new class of monoterpene structure. Organic Letters 2009, 11, 2683-2685) as the compound 2-(1,5,5-trimethylcyclopent-2-en-1-yl)ethyl acetate, in populations of the species collected in the United States. However, this substance turned out to be ineffective in attracting populations of *P. longispinus* in persimmon cultivation in the Valencian Community. Preliminary tests carried out by the Universitat Politècnica de València (UPV) showed that traps baited with virgin females of *P. longispinus* captured 100 times more males than traps baited with the synthetic pheromone described by Millar. According to this result, the most likely hypothesis was that the pheromone complex of the *P. longispinus* populations present in Spain was different from that of the American populations sampled in 2009. This is not uncommon, and this phenomenon has been described for the mealybug *P. ficus,* whose Californian populations produce a single-component pheromone, while Israeli populations produce an additional compound (Zada, A., Dunkelblum, E., Assael, F., Harel, M., Cojocaru, M. & Mendel, Z. (2003) Sex pheromone of the vine mealybug, Planococcus ficus in Israel: occurrence of a second component in a mass-reared population. Journal of Chemical Ecology, 29, 977-988).

In accordance with all of the above, the pheromone described so far for *P. longispinus* was not effective. Therefore, there is a need for new compounds that allow the control and specific monitoring of these populations of *P. longispinus,* and that, in addition to being effective, allows their use in control methods that are environmentally sustainable.

### Brief description of the invention

The present invention solves the problems described in the state of the art since it provides attractant compounds, compositions and devices that comprise said compounds for insects of the species *Pseudococcus longispinus.*

Thus, in a first aspect, the present invention relates to a compound of formula I (hereinafter "compound of the invention"), 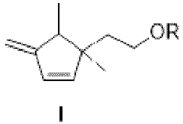 its stereoisomers, 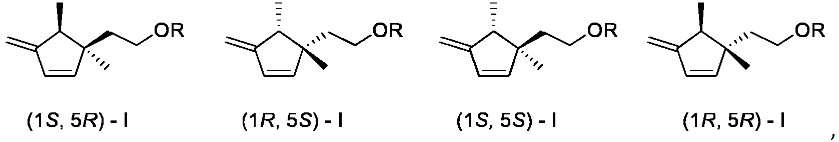 and mixtures thereof;
wherein R is a group -C(=O)R'; being R' selected from C1-C6 alkyl or C3-C6 cycloalkyl.

In a second aspect, the present invention relates to a composition (hereinafter "composition of the invention") comprising the compound of the invention. Particularly, said composition is useful for attracting insects of the species *Pseudococcus longispinus.*

In a third aspect, the present invention relates to a carrier containing the compound or composition of the invention.

In a fourth aspect, the present invention relates to an attractant device for *Pseudococcus longispinus* comprising a carrier containing the compound of the invention or the composition of the invention.

In a fifth aspect, the present invention refers to a method of control and/or monitoring of *Pseudococcus longispinus* populations comprising the use of the compound of the invention or the composition of the invention.

In a further aspect, the present invention relates to the use of a compound or composition of the invention for controlling and/or monitoring of *Pseudococcus longispinus* populations.

Lastly, the present invention relates to a method for preparing the compound of the invention.

### Brief description of figures

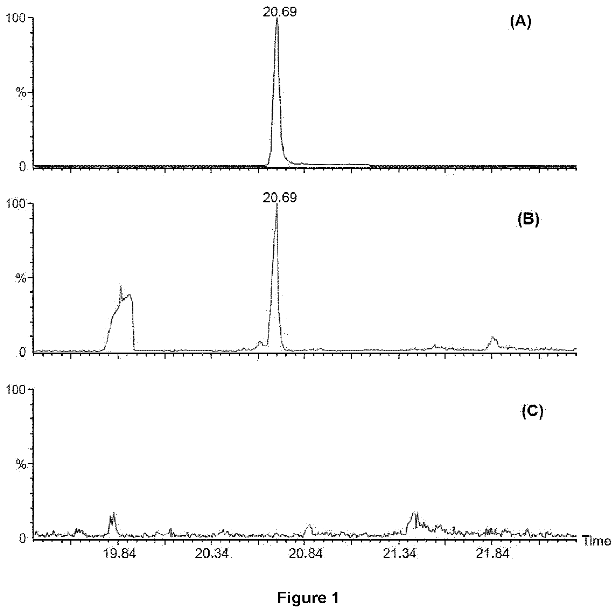

**Figure 1** shows the detection of 2-(1,5-dimethyl-4-methylenecyclopent-2-en-1-yl)ethyl acetate in volatile samples of *Pseudococcus longispinus* individuals reared in the laboratory on lemons. GC traces of (A) synthetic 2-(1,5-dimethyl-4-methylenecyclopent-2-en-1-yl)ethyl acetate as racemate, (B) sample of volatiles from virgin females, (C) sample of volatiles from mated females. The peak with a retention time of 20.69 min detected in the samples of virgin females (B), does not appear in the samples of mated females (C), while it matches the synthetic sample of 2-(1,5-dimethyl-4-methylenecyclopent-2-en-1-yl)ethyl acetate in trace (A).

### Detailed description of the invention

As mentioned above, a first aspect of the invention refers to a compound of formula I, 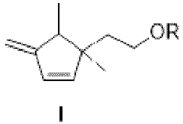 its stereoisomers, 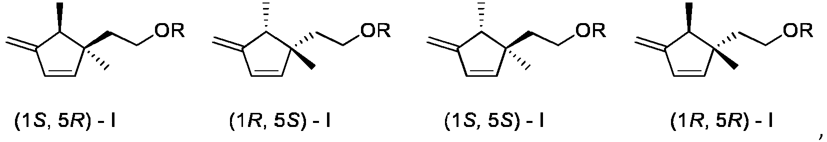 solvates and mixtures thereof;
wherein R is a group -C(=O)R'; being R' selected from C1-C6 alkyl or C3-C6 cycloalkyl.

Compounds of formula I are esters derived from the 2-(1,5-dimethyl-4-methylenecyclopent-2-en-1-yl)ethyl core.

Said compounds of formula I may exist as pure stereoisomers or mixtures of stereoisomers in any ratio, for example, they may have an absolute *R, S* configuration (assigned according to the known Cahn-Ingold-Prelog rules) at carbon atoms 1 and 5 of the 5-membered ring. In particular, the substituted cyclopentenyl ring of the compounds of formula I is characterized by two stereogenic centers, therefore 4 stereoisomers may exist, the structure of which is drawn below: 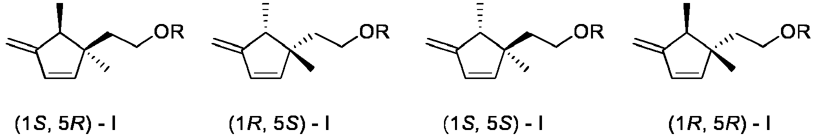 wherein R is defined throughout the description.

In an embodiment, the compounds of formula I encompass the 4 possible stereoisomers in all possible molar ratios between them. In a particular embodiment, the compounds of formula I are the pair of enantiomers (1R, 5R)-I and (1S, 5S)-I. In another particular embodiment, the compounds of formula I are the pair of enantiomers (1S, 5R)-I and (1R, 5S)-I.

The R group of the side chain of the compounds of formula I may encompass various optionally substituted acyl groups. Bioisosteric replacements of the -OC(=O)R' groups may be also acceptable as a skilled person would understand that such replacements would not modify the biological activity of the compounds of formula (I) (J. Med. Chem. 1989,32, 2282). Particular bioisosteric fragments for esters groups include, but are not limited to, amides, ketones, oximes, oxazoles, triazoles, and oxaimidazoles.

In one embodiment, the acyl group of the side chain of the compounds of formula (I) is a group -C(=O)R', wherein R' is selected from C1-C6 alkyl or C3-C6 cycloalkyl. In this context, the term "side chain" denotes the substituent CH₂CH₂OR of the cyclopentenyl ring.

The C1-C6 alkyl or C3-C6 cycloalkyl groups may be optionally substituted. The substitution can occur at one or more C-H positions of the R' group and, unless otherwise indicated, these substituents at each substitution site are independently selected from C1-C4 alkyl, C3-C6 cycloalkyl or halogen.

As used in this document, the following terms have the following definitions:
A halogen denotes fluorine, chlorine, bromine or iodine, preferably fluorine or chlorine.

A "C1-C6 alkyl" group indicates a branched saturated aliphatic or straight chain hydrocarbon of 1 to 6 carbon atoms. Typical alkyl groups include methyl, ethyl, propyl, isopropyl, n-butyl, t-butyl, 2-butyl, pentyl, and hexyl. Similarly, a C1-C4 alkyl group refers to an alkyl having 1 to 4 carbon atoms.

A "C3-C6 cycloalkyl" denotes a non-aromatic partially or fully saturated cyclic aliphatic hydrocarbon group containing 3 to 6 carbon atoms. Examples of C3-C6 cycloalkyl groups include cyclopropyl, cyclobutyl, cyclopentyl, and cyclohexyl.

In a particular embodiment, the group R' is selected from C1-C4 alkyl or C3-C6 cycloalkyl. In a more particular embodiment, the group R' is selected from C1-C4 alkyl or cyclopropyl.

In a preferred embodiment, the R' group is a C1-C4 alkyl; preferably, it is methyl, ethyl, propyl, butyl or isobutyl; more preferably R' is methyl or ethyl.

In a more particular embodiment, the compound of formula I is selected from 2-((1R,5R)-1,5-dimethyl-4-methylenecyclopent-2-en-1-yl)ethyl acetate with chemical structure: 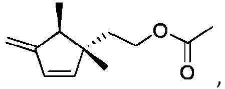 2-((1S,5S)-1,5-dimethyl-4-methylenecyclopent-2-en-1-yl)ethyl acetate with chemical structure: 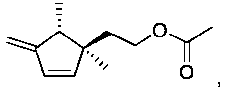 2-((1R,5R)-1,5-dimethyl-4-methylenecyclopent-2-en-1-yl)ethyl propionate with chemical structure: 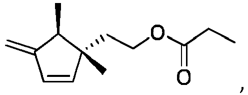 2-((1S,5S)-1,5-dimethyl-4-methylenecyclopent-2-en-1-yl)ethyl propionate with chemical structure: 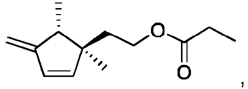 or a mixture thereof.

In another particular embodiment, the compound of formula I is selected from 2-((1R,5S)-1,5-dimethyl-4-methylenecyclopent-2-en-1-yl)ethyl acetate with chemical structure: 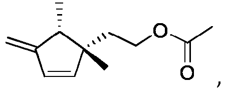 2-((1S,5R)-1,5-dimethyl-4-methylenecyclopent-2-en-1-yl)ethyl acetate with chemical structure: 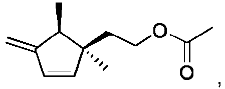 2-((1R,5S)-1,5-dimethyl-4-methylenecyclopent-2-en-1-yl)ethyl propionate with chemical structure: 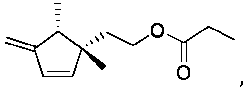 2-((1S,5R)-1,5-dimethyl-4-methylenecyclopent-2-en-1-yl)ethyl propionate with chemical structure: 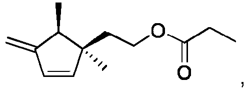 or a mixture thereof.

The compounds of formula (I) may exist as pairs of enantiomers and/or diastereomers. The mixture of two enantiomers with absolute configuration 1R,5R and 1S,5S may comprise the two enantiomers in different ratios between each other, such as from 99:1 to 1:99, from 90:10 to 10:90, from 75:25 to 25:75 or 50:50, respectively (the ratios are expressed as molar ratios). In a preferred embodiment, the mixture of compounds of formula (I) comprises a racemic mixture (50:50 ratio of two enantiomers). In another embodiment, the mixture of two enantiomers with absolute configuration 1R,5R and 1S,5S comprises the two enantiomers in any ratio other than 50:50, such as from 99:1 to 1:99, from 90:10 to 10:90, or from 75:25 to 25:75, respectively. In a further embodiment, the compounds of formula (I) consist of enantiomers with absolute configuration 1R,5R and 1S,5S and in racemic form.

In yet another embodiment, the compounds of formula (I) consist of the mixture of two enantiomers with absolute configuration 1R,5S and 1S,5R, preferably in a ratio of 50:50 or, alternatively, in any other ratio such as from 99:1 to 1:99, from 90:10 to 10:90, or from 75:25 to 25:75, respectively.

The ratio (expressed as molar ratio) between diastereomers may also vary from 99:1 to 1:99, from 90:10 to 10:90, from 75:25 to 25:75 or 50:50. Preferably, the diastereomeric ratio varies from 99:1 to 75:25; even more preferably, such ratio is about 90:10.

In a particular embodiment of the first aspect of the invention, the compounds of formula (I) refers to those compounds where R is a group -C(=O)R', being R' selected from methyl and ethyl, and wherein the stereoisomers are 1R,5R and 1S,5S in a 50:50 molar ratio.

In another particular embodiment of the first aspect of the invention, the compounds of formula (I) refers to those compounds where R is a group -C(=O)R', being R' selected from methyl and ethyl, and wherein the stereoisomers are 1S,5R and 1R,5S in a 50:50 molar ratio.

In a second aspect, the present invention relates to a composition comprising one or more compounds of the present invention, preferably one compound of formula I. Said composition may find use in attracting, controlling and/or monitoring populations of *Pseudococcus longispinus.*

In a more particular aspect, the composition of the present invention comprises an effective amount of the compound of the invention or a mixture of compounds of the invention comprised between 0.001 mg to 100 g. Preferably, the effective amount is in the range of 0.001 mg to 1 g; more preferably in the range of 0.001 to 100 mg. Effective amount is defined as the amount of a compound of formula I or mixtures thereof that acts, alone or in combination with other components in a composition, upon the *Pseudococcus longispinus* and effectively controls the pest caused by said species.

Alternatively, the weight% of the compound(s) of formula I comprised in the composition is between 0.001 and 99.9 wt.% compared to the total weight of the composition; preferably between 0.01 and 99.0 wt.%, even more preferably between 0.1 and 95 wt.%.

As a person skilled in the art is aware of, the amount of compound is variable depending on the type of area, zone or object to be treated, the environmental conditions and the number of days of attraction required, as well as the particular proportion of enantiomers present in the compound of the invention used.

In a more particular aspect, the composition of the present invention may further comprise at least one additional ingredient.

In the present invention, the term "ingredient" refers to any active substance that is incorporated into the composition to perform at least one specific function.

More particularly, the ingredient is selected from antioxidants, radiation protectants, insect control agents, pheromones, and mixtures thereof.

In the present invention, the term "antioxidant" refers to any substance capable of retarding or preventing the oxidation of one or more components of the composition of the invention. Preferably, the antioxidant agent of the composition of the invention is selected from ascorbic acid, erythorbic acid, sodium ascorbate, calcium ascorbate, butylated hydroxyanisole (BHA), butylated hydroxytoluene (BHT), sulfur dioxide, sodium erythorbate, ascorbyl stearate, propyl gallate, octyl gallate, dodecyl gallate, sodium hydrosulfite, lecticin, ascorbyl palmitate, tert-butylhydroquinone (TBHQ) and natural and/or synthetic tocopherols and any combination of the above antioxidants.

In a more particular embodiment, in the attractant composition of the present invention, the antioxidant agent is present in a ratio comprised between 1:1000 and 1:20 by weight with respect to the weight content of compound of formula I. Preferably, the antioxidant agent is present in a ratio of 1:100 by weight with respect to the content by weight of the compound of formula I.

In the present invention, the term "UV radiation protectors" refers to any compound capable of protecting and preserving one or more components of the composition of the present invention from solar degradation. Preferably, the UV radiation protectors of the present invention refer to PABA derivatives, salicylates, cinnamates, benzophenones, benzimidazoles, anthralinates, terpene derivatives, inorganic oxides, and any combination of the above UV protectors. They preferably refer to 4-aminobenzoic acid, 4-hydroxybenzophenone, 2-ethylhexyl salicylate, 2-ethylhexyl trans-4-methoxycinnamate, ethylhexyl 2-cyano-3,3-diphenylacrylate, titanium oxide and/or zinc oxide and any combination of previous UV protectants.

In a particular embodiment, the UV radiation protector comprised in the attractant composition of the present invention is present in a ratio of between 1:200 and 1:20 by weight with respect to the content by weight of the compound of formula I. Preferably, the UV radiation protector is present in a ratio of 1:50 by weight with respect to the content by weight of the compound of the invention.

In the present invention, the term "insect control agent" refers to a chemical substance that causes the death of the insect. More particularly, it refers to insecticides, more particularly it refers to organochlorine, organophosphate, carbamate, pyrethroid, neonicotinoid, tetramic acid, biorational insecticides and combinations thereof.

In the present invention, a pheromone is defined as any substance that an animal secretes and that causes a specific reaction or behavior in a member of the same species, and particularly, a sexual pheromone is defined as that which one of the genera of the species secretes and causes an attraction and copulation response in the other. On the other hand, the term "kairomone" refers to any substance that an organism secretes and intervenes in the communication between individuals of different species, benefiting the organism that receives it.

In a more particular aspect, the composition of the invention comprises at least one agriculturally acceptable excipient.

In the present invention, the term "agriculturally acceptable excipient" refers to any substance that is incorporated into the composition of the present invention to provide shape, consistency, flavor, odor, color, etc. More particularly, the excipient is selected from among binders, diluents, solvents, disintegrants, lubricants, colorants, sweeteners, flavors, preservatives, and mixtures thereof.

In a particular aspect, the composition of the invention is presented in a formulation selected from emulsion, solution, dispersion, aerosol, liquid, gel, powder, granule, paste and tablet. Preferably, the formulation is selected from emulsion, solution, aerosol, or tablet.

The compound and the composition of the invention as previously defined may be incorporated into a carrier, which is a further aspect of the invention.

In the context of the present invention, the term "carrier" refers to a substrate or matrix capable of carrying or containing the compound of the present invention or the composition of the present invention. More particularly, the carrier is selected from a polymeric matrix, wood, ceramic, metal, leather, nylon, rubber, paraffin, wax, cotton, foam, textile material, granules, polymers, silicas, resins, and adhesive tapes, among others.

In a more particular aspect, the compound of the present invention or the composition of the present invention is deposited, absorbed, adsorbed, pulverized or coated or is found in any physical or chemical way that allows it being incorporated into the carrier, depending on the nature of the compound, composition and carrier. When the compound or mixture of compounds of the invention is incorporated in the carrier, the weight % of the compound(s) of formula I comprised in the carrier is between 0.001 and 99.9 wt.% compared to the total weight of the carrier; preferably between 0.01 and 99.0 wt.%, even more preferably between 0.1 and 95 wt. %.

In a fourth aspect, the present invention relates to an attractant device for *Pseudococcus longispinus* comprising a carrier containing the compound or the composition of the invention as defined in the embodiments above. In a particular embodiment, the device of the present invention comprises a trap.

In the present invention, the term "trap" refers to any device that traps and/or affects insects and/or retains them. In a particular embodiment, the trap comprises a toxic or pathogenic substance for the target insect. In a more particular embodiment, the trap is a surface that comprises an adhesive.

In a particular embodiment, the carrier of the device of the present invention is contained in the trap.

In a particular embodiment, the carrier of the device of the present invention is separate from the trap.

A certain amount of the compound of formula I is emitted from said attractant device causing populations *Pseudococcus longispinus* to be attracted into the device. The effective average emission of the compound of formula I is at least 0.1, at least 0.5, at least 1.0, at least 2.0, at least 5.0 µg/day. In another embodiment, the effective average emission of the compound of formula I is comprised between 0.1 and 500 µg/day. In another embodiment, the effective average emission of the compound of formula I is comprised between 0.1 and 250 µg/day. In a preferred embodiment, the effective average emission of the compound of formula I is comprised between 0.1 and 100 µg/day, even more preferably between 0.1 and 50 µg/day. Alternatively, the effective average emission of the compound of formula I may be at least 0.001, at least 0.01, at least 0.1, at least 1.0, at least 10 µg/day/ha (where "ha" refers to 1 hectare). In another embodiment, the effective average emission of the compound of formula I is comprised between 0.001 and 500 µg/day/ha. In another embodiment, the effective average emission of the compound of formula I is comprised between 0.01 and 250 µg/day/ha. In a preferred embodiment, the effective average emission of the compound of formula I is comprised between 0.01 and 100 µg/day/ha, even more preferably between 0.1 and 50 µg/day/ha. In the present invention, "effective average emission" relates to the emission flow of at least one compound of formula I that is released at a substantially constant speed, which acts upon the *Pseudococcus longispinus* by attracting, catching, capturing, mating disruption and/or by death of male individuals belonging to said species.

The present invention also refers to a method of control and/or monitoring of populations of *Pseudococcus longispinus* (hereinafter, the method of the present invention) that comprises the use of the compound, of the composition or of the device of the present invention as defined above. More particularly, the method of the present invention comprises the use of the device of the present invention. Thus, the method of the invention involves an effective average emission of one or more compounds of formula I as specified above.

In a particular embodiment, the control and/or monitoring method is carried out by attracting male individuals belonging to the species *Pseudococcus longispinus.*

In another particular embodiment, the control and/or monitoring method is carried out through mating disruption of male individuals belonging to the species *Pseudococcus longispinus.*

In another particular embodiment, the control and/or monitoring method is carried out by means of the death of male individuals belonging to the species of *Pseudococcus longispinus.*

In an additional aspect, the present invention relates to the use of a compound or composition or device of the invention for control and/or monitoring of *Pseudococcus longispinus* populations. Particularly, the invention relates to the use of an "effective average emission" of a compound of the invention comprised in the composition or device of the invention for control and/or monitoring of *Pseudococcus longispinus* populations. Such effective average emission is defined as above.

Lastly, in a further aspect the present invention also relates to a method for preparing a compound of the invention of formula I (drawn above) comprising the steps of:
i. aldol condensation of a compound of formula **1:** 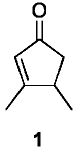 with p-formaldehyde to obtain a compound of formula **2:** 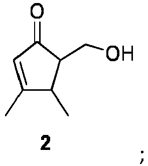
ii. Protecting the hydroxyl group of compound **2** with an hydroxyl protecting group PG₁;
iii. reduction of the carbonyl of compound **2** and protection of the resulting -OH group with a hydroxyl-protecting group PG₂ to obtain a compound of formula **3:** 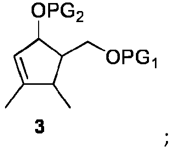
iv. rearrangement of compound **3** into compound **4:** 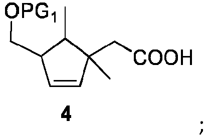
v. reduction of the carboxyl group of the compound **4** and protection of the resulting hydroxyl group with a acetyl group to obtain compound **5:** 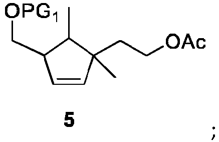
vi. deprotection of the PG1-protected hydroxyl and iodination of the deprotected compound to obtain compound **6:** 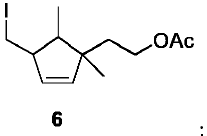
vii. dehydrohalogenation of compound **6** to obtain a compound of formula I, wherein R = Ac, or dehydrohalogenation of compound **6** followed by hydrolysis of the acetyl group and reaction with an anhydride of formula 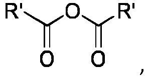 wherein R' selected from C2-C6 alkyl or C3-C6 cycloalkyl to obtain a compound of formula I, wherein R ≠ Ac.

All steps in the method above may be performed in solvents or using neat reagents, preferably, all reagents are dissolved in an organic solvent and the reactions are carried out under stirring until no more conversion is observed.

In step i), the aldol condensation is preferably conducted in presence of a base. Suitable bases can be organic or inorganic bases; in a particular embodiment the base is selected from potassium t-butoxide, potassium hydroxide, sodium hydride, lithium bis(trimethylsilyl)amide, lithium diisopropylamide, and methyl lithium. Preferably the base is lithium bis(trimethylsilyl)amide. The aldol condensation is also carried out at low temperature so side-reactions are minimized. The temperature in step i) may be comprised between -50 °C and 0 °C, preferably between -40 °C and -20 °C, even more preferably at 30 °C.

In step ii), a hydroxyl protecting group is used to form a -CH₂OPG, protected group. Introduction of protecting groups belongs to the common knowledge of a skilled person and one would immediately find suitable reagents and conditions for this step (see Peter G. M. Wuts, Theodora W. Greene, Greene's Protective Groups in Organic Synthesis, Fourth Edition, DOI:10.1002/0470053488). Among the preferred hydroxyl protecting groups are methoxymethyl ether (MOM), tetrahydropyranyl ether (THP), t-butyl ether, allyl ether, benzyl ether, t-butyldimethylsilyl ether (TBDMS), t-butyldiphenylsilyl ether (TBDPS), acetic acid ester, pivalic acid ester, benzoic acid ester; preferably THP is used as PG₁. A catalytic amount of an acid may be required to carry out the protection step i), preferably p-TsOH is used as acid catalyst.

In step iii), the reduction of the carbonyl group of compound **2** is performed in presence of a metal hydride and a temperature comprised between -10 and 10 °C, more preferably at about 0 °C. Several metal hydrides may be suitable for this step such as alkali hydrides, however, LiAlH₄ is preferred among them. Protection of the resulting alcohol or alcoholate is again performed with a suitable hydroxyl protecting group to generate a - OPG₂ group in the corresponding compound **3.**

Next, compound **3** is subjected in step iv) to an Ireland-Claisen rearrangement skeletal rearrangement in presence of TBDMSCI and a base, wherein preferably the base is an organolithium compound, more preferably LDA. Carboxylic acid **4** is obtained.

In step v), the reduction of carboxylic acid **4** to compound **5** is performed in presence of a metal hydride, preferably lithium aluminium hydride, and in an aprotic solvent. The alcohol or alcoholate product is protected with an acetyl group by employing acetic anhydride as acetyl precursor; however other acetyl precursors are known and may be employed.

Step vi) is performed by deprotecting the acetate **5** in an organic solvent or in a mixture of organic solvents in presence of a catalytic amount of acid; preferably the acid is p-TsOH. The amount of acid may vary between 0.01 and 10 mol%, preferably between 0.1 and 5 mol%, even more preferably the amount of acid is about 1 mol%. The iodination of the protected alcohol is performed under Appel conditions in presence of a triarylphosphine, preferably PPh₃, an iodination agent, preferably I₂, and a base, preferably, imidazole.

Finally, in step yii) the iodo-derivative **6** is subjected to dehydrohalogenation in presence of a base, preferably a N-heterocyclic base such as DBU, optionally heating the mixture at a temperature between 40-80 °C, more preferably at about 60 °C. A compound of formula I, wherein R = Ac is then obtained. Alternatively, the dehydrohalogenation of compound 6 followed by hydrolysis of the acetyl group and reaction with an anhydride of formula: 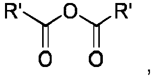 wherein R' selected from C2-C6 alkyl or C3-C6 cycloalkyl, leads to a compound of formula I, wherein R ≠ Ac.

### Examples

### Abbreviations

Ac: acetyl
PG: protecting group
GC-MS: gas chromatography-mass spectroscopy
LDA: lithium diisopropylamide
TBDMSCI: tert-butyldimethylsilyl chloride
HMDSLi: lithium bis(trimethylsilyl)amide
p-TsOH: p-toluenesulfonic acid
DCM: dichloromethane
THF: tetrahydrofuran

### Example 1: Detection of 2-(1,5-dimethyl-4-methylenecyclopent-2-en-1-yl)ethyl acetate in samples from virgin females of Pseudococcus longispinus by means of volatile collection techniques.

To capture the volatiles emitted by *Pseudococcus longispinus* females in different mating status, individuals from the laboratory colony maintained on organically grown lemons at the Center for Agricultural Chemical Ecology (CEQA, Universitat Politècnica de València, Valencia) were used. The insect colony was kept in a chamber under controlled conditions, at a temperature of 23 ± 2 °C and 60-70% relative humidity. Sampling of the volatiles emitted by the insects was carried out by aerating the individuals and capturing the effluvia in glass cartridges filled with the Porapak-Q adsorbent matrix.

Groups of 200-300 individuals were placed on the rearing substrate in 5-L glass containers, through which a filtered air flow of 0.3 L/min was passed. Every 7-8 days, the adsorbent material was washed with 20 mL of pentane to elute the captured substances. The eluents were analyzed by gas chromatography coupled with mass spectrometry (GC-MS). Chromatographic analysis was performed on a Clarus 600 GC-MS equipment (PerkinElmer Inc.), equipped with a ZB-5MS capillary column (30 m × 0.25 mm i.d. × 0.25 µm; Phenomenex Inc.) and the following temperature programming: 40 °C for 2 min; 5 °C/min to 180 °C and then increase to 280 °C at 10 °C/min, holding at 280 °C for 1 min. Helium was used as carrier gas with a flow of 1 mL/min. The detection was carried out in electronic impact mode (70 eV) and the temperature of the ionization source and the transfer line were 200 °C and 250 °C, respectively. Once an exclusive peak of the samples of virgin females was detected, the corresponding compound was isolated from the eluted mixture by gravity chromatography of the total extract with pentane:diethyl ether mixtures (100:0, 95:5, 80:20, 0:100) as eluents. After isolation, the structural elucidation of the compound was carried out with the data provided by the GC-MS and nuclear magnetic resonance (NMR) (Bruker, 600 MHz). Finally, the spectra of the natural substance were compared with those of a sample synthesized by Ecologia y Protección Agricola SL (Carlet, Valencia).

The chromatographic analysis revealed one peak that appeared exclusively in the samples of volatiles emitted by virgin females and not in those of females that had mated or immature individuals, as can be seen in Figure 1. This peak corresponded to 2-(1,5-dimethyl-4-methylenecyclopent-2-en-1-yl)ethyl acetate, as identified by spectrometric data, and later confirmed by comparison with a synthetic sample produced in the laboratory as described in Example 2. This is a new and different substance from that described by Millar et al. in 2009.

### Example 2: Synthesis of 2-(1,5-dimethyl-4-methylenecyclopent-2-en-1-yl)ethyl acetate

The synthesis of 2-(1,5-dimethyl-4-methylenecyclopent-2-en-1-yl)ethyl acetate has been carried out according to scheme 1 shown below.

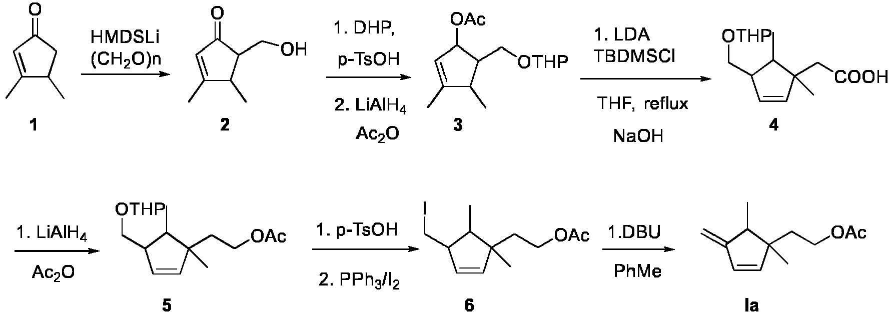

Note: The following procedure affords two diastereomers of **la** in 9:1 ratio approximately as per NMR analysis, however, for the sake of clarity, only ¹H and ¹³C NMR signals of the major diastereomer are given below.

Ketone **1** (5g, 0.045 mol) was prepared according to Leviredend, M.L; Conia, Sur J.M., Sur la preparation de cyclopenténones par action de l'acide phosphorique sur les esters d'acides éthyléniques, Bulletin de la societé quimique de france, 8-9, 1970 (and references therein). The ketone **1** was added dropwise to a HMDSLi solution (65 mL, 0.9 M) in THF at - 30°C, over 90 min. The solution was warmed up to 0 °C, and p-formaldehyde (4.05 g, 3 eq) was added to the solution in one portion. After 30 min, the solution was poured over saturated ammonium chloride solution (45 ml) and extracted three times with ethyl acetate (30 mL). The combined organic phases were subsequently washed with solutions of HCl (1M), NaHCO₃ (1M), brine and dried over anhydrous MgSO₄. After solvent evaporation, the crude material was purified by using column chromatography (Silica gel, Hexane:AcOEt 8:2) to afford alcohol **2** (2.5 g, 40 % yield). ¹H NMR (400 MHz, CDCl₃) δ_{H} 5.41 (1H, m), 3.92-3.72(2H, m), 2.65 (1H, m), 1.70 (3H, m), 1.71-1.66 (1H, m), 1.05 (3H, d, J 7.2 Hz); ¹³C NMR (100 MHz) 210.2, 173.2, 127.8, 61.5, 57.3, 46.0, 18.2, 14.3. MS (70eV) m/z: 53 (3), 67 (2), 81 (8), 94 (7), 95 (7), 107 (1), 109 (7), 110 (4), 122 (4), 125 (2), 140 (7, M+).

Alcohol **2** (2.5 g, 0.018 mol) was dissolved in DCM (15 mL) and cooled to 0 °C. 3,4-Dihydropyran (DHP) (1.89 mL, 0.021 mol) and a catalytic amount of p-TsOH (0.015 eq) were subsequently added to the solution. The reaction was warmed up to room temperature and was monitored by TLC, and after 3 h of continuous stirring, the solution was subsequently washed with NaHCO₃ (1M), brine and dried over anhydrous MgSO₄. After solvent evaporation, the crude mixture of hydroxyl protected compound **2** was used in the next step without further purification. Crude hydroxyl-protected compound **2** was dissolved in anhydrous Et₂O (7 mL) and dropwise added to a 0 °C cooled suspension of LiAlH₄ (0.021 mol, 0.8 g) in anhydrous Et₂O (25 mL) under inert atmosphere (N₂). The suspension was kept at this temperature while stirring for additional 1.5 hours. After this period, acetic anhydride (0.042 mol, 1.96 mL) was added and the reaction was warmed up to room temperature. The reaction was monitored by TLC and after 15 h of continuous stirring, a saturated solution of ammonium chloride (5 mL) was slowly added while the solution was vented with an N₂ stream. The mixture was poured in ethyl acetate (30 mL) and the water phase separated. The organic layers were subsequently washed with solutions of HCl (1M), NaHCO₃ (1M), brine and dried over anhydrous MgSO₄. After solvent evaporation, acetate **3** was purified by using column chromatography (Silica gel, Hex:AcOEt 9:1) to yield a pale yellow oil (3.93 g, 70 % yield over two steps). ¹H NMR (400 MHz, CDCl₃) δ_{H} 5.47 (1H, m), 5.36 (1H, m), 4.6 (1H, m), 3.84-3.77 (2H, m), 3.56-3.45 (2H, m), 2.31 (1H, m), 2.01 (3H, s), 2.02-1.98 (1H, m), 1.85-1.75 (1H, m), 1.71 (3H, s), 1.70-1.49 (6H, m), 1.14 (3H, d, J 7.2 Hz); ¹³C NMR (100 MHz) 171.2, 151.7, 122.7, 98.9, 81.6, 68.7, 62.1, 53.1, 45.2, 30.7, 25.6, 21.5, 19.8, 19.1,15.01. MS (70eV) m/z 51 (40), 43 (55), 55 (20), 57 (22), 60 (10), 65 (10), 67 (25), 77 (20), 79 (20), 85 (100), 91 (40), 93 (25), 95 (20), 106 (28), 107 (28), 109 (25), 124 (10), 141 (10), 183 (2), 208 (1), 226 (1).

A solution of acetate **3** (2.5 g, 9.3 mmol) in dry THF (10 mL) was added dropwise to a LDA solution (11.9 mL, 0.9 M) in THF at - 78°C, over 60 min. Chloro *tert*-butyldimethyl silane (11.1 mmol, 1.68 g, 1.2 eq) in dry THF (6 mL) was added to the solution in one portion. The solution was warmed up to room temperature during 3 h and then refluxed for 24 h. After cooling down to room temperature, NaOH solution (2M, 20 mL) was added and the resultant biphasic mixture was stirred for 1h. The solution was acidified to pH 4-5 with an aqueous citric acid solution (20 %) and extracted three times with ethyl acetate (25 mL). The organic layers were subsequently washed with solutions of HCl (1M), NaHCO₃ (1M), brine and dried over anhydrous MgSO₄. After solvent evaporation, crude residue was purified by using column chromatography (Silica gel, Hex:AcOEt 8:2) to give acid **4** as an oil (1.2 g, 48 % yield). ¹H NMR (400 MHz, CDCl₃) δ_{H} 5.87 (1H, dd, J 5.9, 2.5 Hz), 5.74 (1H, dt, J 22, 2.5 Hz), 4.61 (1H, ddd, J 13.5, 4.2, 2.8 Hz), 3.95-3.80 (1H, m), 3.80-3.58 (1H, m), 3.65-3.50 (1H, m), 3.45-3.30 (1H, m), 2.65-2.52 (1H, m), 2.35-2.2 (2H, m), 1.85-1.75 (1H, m), 1.73-1.50 (8H, m), 1.24 (3H, s), 1.05 (3H, d, J 7.2 Hz); ¹³C NMR (100 MHz) 177.7, 139.7, 131.8, 99.3, 70.9, 62.3, 51.9, 48.9, 40.6, 30.7, 29.3, 25.6, 24.1, 19.6, 12.7. MS (70eV) m/z 55 (3), 67 (3), 79 (2), 85 (36), 91 (3), 107 (9), 121 (3), 166 (4), 238 (0.1, M+).

A solution of acid **4** (1.2 g, 4.5 mmol) in anhydrous THF (6 mL) and dropwise added to a 0 °C cooled suspension of LiAlH₄ under inert atmosphere (N₂). The suspension was kept at this temperature with continuous stirring for 1,5 h. After this period, acetic anhydride (10 mmol, 0.43 mL) was added and the reaction was warmed up to room temperature and monitored by TLC. After 15 h of continuous stirring, a saturated solution of ammonium chloride was slowly added while the solution was vented by using an N₂ stream. The mixture was extracted three times with ethyl acetate (20 mL). The organic layers were subsequently washed with solutions of HCl (1M), NaHCO₃ (1M), brine and dried over anhydrous MgSO₄. After solvent evaporation, the crude material was purified by using column chromatography (Silica gel, Hexane:AcOEt 9:1) to give acetate **5** as an oil (0.86 g, 65 % yield over two steps). ¹H NMR (400 MHz, CDCl₃) δ_{H} 5.74-5.5 (2H, m), 4.61 (1H, ddd, J 10.8, 3.2, 3.2 Hz), 4.15-3.90 (2H, m), 3.91-3.80 (1H, m), 3.79-3.55 (1H, m), 3.55-3.45 (1H, m), 3.43-3.25 (1H, m), 2.62-2.50 (1H, m), 2.03 (3H, s), 1.85-1.75 (1H, m), 1.73-1.48 (8H, m), 1.07 (3H, s), 1.03 (3H, d, J 7.2 Hz); ¹³C NMR (125 MHz) 171.3, 139.7, 131.8, 99.3, 70.5, 62.3, 62.1, 52.3, 48.8, 48.7, 48.5, 34.9, 30.8, 25.6, 21.2, 19.7, 12.6. MS (70eV) m/z 43 (6), 55 (3), 57 (3), 67 (2) 77(1), 85 (37), 91 (3), 93 (3), 107 (6), 119 (2), 121 (1), 134 (5), 194 (1), 266 (0.1).

Acetate **5** (0.86 g, 2.92 mmol) was dissolved in a DCM/MeOH mixture (8 mL; 1:1), and a catalytic amount of p-TsOH (0.01 eq) was added to the solution. The reaction was monitored by TLC and after 3 hours of continuous stirring, the solution was poured in 10 mL of DCM and subsequently washed with NaHCO₃ (1M), brine and dried over anhydrous MgSO₄. After removal of the solvent under reduced pressure, the crude material was dissolved in anhydrous DCM (15 mL) and PPh₃ (1.08 g, 2.92 mmol), I₂ (1.027 g, 2.92 mmol) and imidazole (0.46 g, 7.3 mmol) were subsequently added. After 3 hours of continuous stirring, the suspension was filtered off to remove the precipitate formed, and subsequently washed with solutions of HCl (1M), NaHCO₃ (1M), brine and dried over anhydrous MgSO₄. After solvent evaporation, acetate **5** was purified by using column chromatography (Silica gel, Hex:AcOEt 9:1) to give iodide **6** as a pale yellow oil (0.85 g, % 90 % yield over two steps). Spectroscopical data: ¹H NMR (400 MHz, CDCl₃) δ_{H} 5.72 (1H, dd, J 5.9, 1.1Hz), 5.62 (1H, dd, J 5.9, 2.0 Hz), 4.18-3.90 (2H, m), 3.46 (1H, dd, J 9.8, 4.1 Hz), 3.07 (1H, dd, J 9.8, 7.2), 2.48 (1H, m), 2.03 (3H, s), 1.65-1.55 (2H, m), 1.09 (3H, s), 1.01 (3H, d, J 7.2 Hz); ¹³C NMR (125 MHz) 171.2, 140.3, 133.1, 62.3, 53.6, 51.5, 49.9, 35.1, 25.8, 21.3, 12.3, 11.7. MS (70eV) m/z 48 (8), 55(2), 67 (1), 79 (3), 91 (6), 93 (10), 108 (13), 119 (1), 135 (18), 235 (5), 247 (0.3), 262 (0.1).

Iodide 6 (500 mg, 1.6 mmol) was dissolved in toluene (12 mL) and DBU (2.1 mmol, 0.31 ml) was added, and the solution was heated to 60 °C for 6 h. After this period, the solution was cooled down to room temperature and poured over hexanes (10 ml ml) and subsequently washed with HCl (1 M), NaHCO₃ (1M), brine and dried over anhydrous MgSO₄. After solvent evaporation, the crude mixture was purified by using column chromatography (Silica gel, Hexane:AcOEt 9:1) to give 2-(1,5-dimethyl-4-methylenecyclopent-2-en-1-yl)ethyl acetate **I** as a pale yellow oil (282 mg, 91 % yield). ¹H NMR (500 MHz, CDCl₃) δ_{H} 6.14 (1H, d, J 5Hz), 5.95 (1H, d, J 5Hz), 4.84 (1H, d, J 3Hz), 4.65 (1H, d, J 3Hz), 4.1-3.94 (2H, m), 2.38 (1H, m), 2.01 (3H, s), 1.65-1.54 (2H, m), 1.12 (3H, s), 1.07 (1H, d, J 7.1 Hz); ¹³C NMR (125 MHz) δ_{C} 171.1, 157.9, 145.1, 132.0, 102.6, 62.2, 49.1, 48.7, 35.9, 25.8, 21.2, 11.8. MS (70eV) m/z: 43 (6), 53 (1), 65 (1), 79 (5), 91 (12), 107 (15), 119 (15), 134 (11), 194 (2, M+).

### Example 3: Synthesis of 2-(1,5-dimethyl-4-methylenecyclopent-2-en-1-yl)ethyl propionate

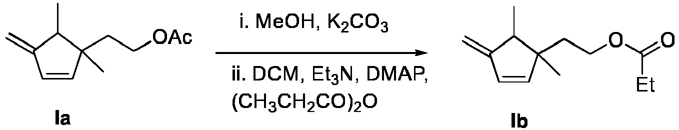

Compound **la** (100 mg, 0.51 mmol) was dissolved in MeOH (5 mL) and K₂CO₃ (207 mg, 3 eq) was added to the solution. After 2 h of continuous stirring, the suspension was filtered off and the solution was poured in ethyl acetate and subsequently washed with HCl (0.1 M), NaHCO₃ (5 %), brine and dried over anhydrous MgSO₄. After solvent evaporation, the crude alcohol was dissolved in anhydrous DCM (5 mL) and Et₃N (0.2 mL, 3eq), 4-dimethylamino pyridine (0.01 eq) and propionic anhydride (0.14 mL, 1.1 eq) were subsequently added. After 3 h of continuous stirring, the solution was transferred to a separation funnel and subsequently washed with HCl (0.1 M), NaHCO₃ (5 %), brine and dried over anhydrous MgSO₄. After solvent evaporation, the crude material was purified by using column chromatography (Silica gel, Hexane:AcOEt 95:5) to give compound **Ib** 2-(1,5-dimethyl-4-methylenecyclopent-2-en-1-yl)ethyl propionate as a pale yellow oil (100 mg, 95 % yield). δ_{H} (400 MHz, C₆D₆) 6.03 (1H, d, J 5.6 Hz), 5.71 (1H, d, J 5.6 Hz), 4.85 (1H, m), 4.67 (1H, m), 4.15-3.94 (2H, m), 2.26 (1H, m), 2.04 (2H, q, 7.6 Hz), 1.54-1.40 (2H, m), 1.00 (3H, t, 7.6 Hz), 0.89 (3H, s), 0.74 (1H, d, J 6.6 Hz); MS (70eV) m/z: 57 (5), 65 (1), 79 (4), 91 (10), 107 (16), 119 (16), 134 (11), 208 (1, M+).

### Example 4: Attraction response tests of Pseudococcus longispinus males in the field

The response of *Pseudococcus longispinus* males to 2-(1,5-dimethyl-4-methylenecyclopent-2-en-1-yl)ethyl acetate was evaluated by means of a field trial carried out in a persimmon orchard, located in the town of Alginet (Valencia, Spain). Six blocks of 3 devices were installed: (A) a device with a carrier, without attractant and with a trap consisting of a white sticky cardboard (95 x 150 mm), (B) a device with a carrier loaded with 250 µg of the described substance by Miller et al. in 2009 (e.g. 2-(1,5,5-trimethylcyclopent-2-en-1-yl)ethyl acetate) and a trap consisting of a white sticky cardboard, and (C) a carrier device loaded with 250 µg of 2-(1,5-dimethyl-4-methylenecyclopent-2-en-1-yl)ethyl acetate and a trap consisting of a white sticky cardboard. Within each block, the devices were placed at a distance of 10 m from each other, while the distance between blocks was at least 30 m. The carriers loaded with the substances were of the rubber septa type and were inserted in the center of the trap.

The captures obtained in each of the traps were reviewed biweekly and the captured individuals were taken to the laboratory to be identified and counted.

The number of males captured per trap and per day was compared using a variance analysis (ANOVA; LSD test for comparison of means, P < 0.05), prior transformation (In(x+1)) of the data in order to homogenize the variance.

The results showed that the devices baited with 2-(1,5-dimethyl-4-methylenecyclopent-2-en-1-yl)ethyl acetate (C) have a significantly higher attractive power than the traps without attractant (A) and that the pheromone described by Millar et al. (B) (Table 1).

The results of the analyses of the content of the attractant composition after 21 days show that the final content of the substance was 134 µg. Therefore, the average emission during those 21 days is 5.52 ± 1.20 µg/day.

**Table 1. Captures (mean and standard error (se)) of P. longispinus males obtained in the field trial conducted in Alginet. Mean values followed by different letters are significantly different (ANOVA, LSD test at P < 0.05).**

| **Device** | **mean** | **se** | **ANOVA** |
|---|---|---|---|
| Blank (A) | 0.01 a | 0.01 | |
| Millar et al. (B) | 0.21 a | 0.15 | F_{2,44} = 199.33 |
| | | | P < 0.0001 |
| Acetate of formula I (C) | 19.35 b | 10.91 | |

A second field trial was conducted to evaluate the attractant activity of 2-(1,5-dimethyl-4-methylenecyclopent-2-en-1-yl)ethyl propionate, in a persimmon orchard located in L'Alcúdia (Valencia, Spain). Four blocks of 3 devices were installed: (A) a device with a carrier, without attractant and with a trap consisting of a white sticky cardboard (95 x 150 mm), (B) a device with a carrier loaded with 250 µg of 2-(1,5-dimethyl-4-methylenecyclopent-2-en-1-yl)ethyl propionate and a trap consisting of a white sticky cardboard, and (C) a carrier device loaded with 250 µg of 2-(1,5-dimethyl-4-methylenecyclopent-2-en-1-yl)ethyl acetate and a trap consisting of a white sticky cardboard. The rest of trial conditions were the same as those described above.

The results showed that the devices baited with 2-(1,5-dimethyl-4-methylenecyclopent-2-en-1-yl)ethyl propionate (B) had a significant attractant activity and captured significantly more males than the traps without attractant (A) (Table 2). Nevertheless, the devices baited with 2-(1,5-dimethyl-4-methylenecyclopent-2-en-1-yl)ethyl acetate (C) achieved the highest level of captures.

**Table 2. Captures (mean and standard error (se)) of P. longispinus males obtained in the field trial conducted in L'Alcúdia. Mean values followed by different letters are significantly different (ANOVA, LSD test at P < 0.05).**

| **Device** | **mean** | **se** | **ANOVA** |
|---|---|---|---|
| Blank (A) | 9.89 a | 5.33 | |
| Propionate of formula I (B) | 89.07 b | 50.45 | F_{2,16} = 561.95 |
| | | | P < 0.0001 |
| Acetate of formula I (C) | 406.58 c | 160.35 | |

## Claims

1. A compound of formula I, 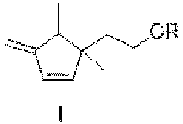 its stereoisomers, 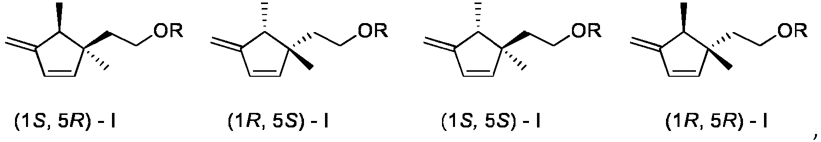 and mixtures thereof;
wherein R is a group -C(=O)R'; being R' selected from C1-C6 alkyl or C3-C6 cycloalkyl.

2. A compound of formula I according to claim 1, wherein R is a group -C(=O)R'; being R' selected from methyl or ethyl.

3. Composition for controlling and/or monitoring of populations of *Pseudococcus longispinus* comprising a compound according to any of claims 1 to 2.

4. Composition according to claim 3 comprising at least an additional ingredient.

5. Composition according to claim 4, wherein the additional ingredient is selected from attractants, antioxidants, radiation protectors, insect control agents, pheromones, kairomones, and mixtures thereof.

6. Composition according to any of claims 3 to 5, comprising at least one agriculturally acceptable excipient.

7. Composition according to claim 6, wherein the agriculturally acceptable excipient is selected from binders, diluents, disintegrants, lubricants, colorants, sweeteners, flavorings, preservatives, and mixtures thereof.

8. Composition according to any of the preceding claims, **characterized in that** the composition is formulated as an emulsion, solution, dispersion, aerosol, liquid, gel, powder, granule, paste or tablet; preferably the composition is formulated as an emulsion, solution, aerosol, or tablet.

9. Carrier containing the compound of formula according to any of claims 1 or 2 or the composition according to any of claims 3 to 8.

10. Attractant device for insects of the species *Pseudococcus longispinus* comprising the carrier according to claim 9.

11. Attractant device according to claim 10, **characterized in that** the effective average emission of the compound of formula I, as defined in claim 1 or 2, or comprised in the composition according to any of claims 3 to 8 or in the carrier according to claim 9, is between 0.1 and 250 µg/day, preferably between 0.1 and 100 µg/day, more preferably between 0.1 and 50 µg/day.

12. Method for controlling and/or monitoring of populations of *Pseudococcus longispinus* comprising the use of the compound according to claims 1 to 2, the composition according to any of claims 3 to 8, or the device according to claims 10 or 11.

13. Method for controlling and/or monitoring of populations of *Pseudococcus longispinus* according to claim 12, where the control is carried out by attracting, catching, capturing by mating disruption and/or by death of male individuals belonging to the species of *Pseudococcus longispinus.*

14. Use of a compound according to any of claims 1 to 2, of a composition according to any of claims 3 to 8, of a carrier according to claim 9 or of a device according to claims 10 or 11 for control and/or monitoring of *Pseudococcus longispinus* populations.

15. Method for the synthesis of a compound according to claim 1 comprising the step of:
i. Aldol condensation of a compound of formula **1:** 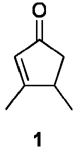 with p-formaldehyde to obtain a compound of formula **2:** 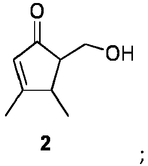
ii. Protecting the hydroxyl group of compound **2** with an hydroxyl protecting group PG₁;
iii. Reduction of the carbonyl of the product from step ii and protection of the resulting -OH group with a hydroxyl-protecting group PG₂ to obtain a compound of formula **3:** 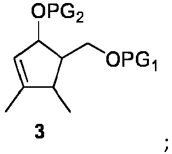
iv. Rearrangement of compound **3** into compound **4:** 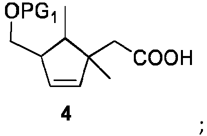
v. Reduction of the carboxyl group of the compound **4** and protection of the resulting hydroxyl group with an acetyl group to obtain compound **5:** 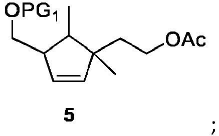
vi. deprotection of the PG1-protected hydroxyl group of compound **5** and iodination to obtain compound **6:** 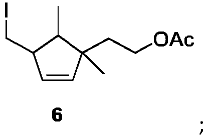
vii. dehydrohalogenation of compound **6** to obtain a compound of formula I, wherein R is Ac, or dehydrohalogenation of compound **6** followed by hydrolysis of the acetyl group and reaction with an anhydride of formula 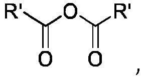 wherein R' selected from C2-C6 alkyl or C3-C6 cycloalkyl to obtain a compound of formula I, wherein R is different from Ac.

## Patentansprüche

1. Eine Verbindung der Formel I, 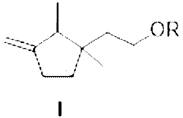 deren Stereoisomere, 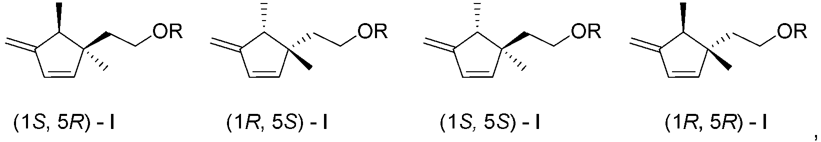 und Gemische davon;
wobei R eine Gruppe -C(=O)R' darstellt; wobei R' ausgewählt ist aus C1-C6-Alkyl oder C3-C6-Cycloalkyl.

2. Eine Verbindung der Formel I gemäß Anspruch 1, wobei R eine Gruppe -C(=O)R' darstellt; wobei R' ausgewählt ist aus Methyl oder Ethyl.

3. Zusammensetzung zur Bekämpfung und/oder Überwachung von *Pseudococcus* longispinus-Populationen, umfassend eine Verbindung gemäß einem der Ansprüche 1 bis 2.

4. Zusammensetzung gemäß Anspruch 3, umfassend mindestens einen zusätzlichen Inhaltsstoff.

5. Zusammensetzung gemäß Anspruch 4, wobei der zusätzliche Inhaltsstoff ausgewählt ist aus Lockstoffen, Antioxidantien, Strahlenschutzmitteln, Mitteln zur Insektenbekämpfung, Pheromonen, Kairomonen und Gemischen davon.

6. Zusammensetzung gemäß einem der Ansprüche 3 bis 5, umfassend mindestens einen landwirtschaftlich verträglichen Exzipienten.

7. Zusammensetzung gemäß Anspruch 6, wobei der landwirtschaftlich verträgliche Exzipient ausgewählt ist aus Bindemitteln, Verdünnungsmitteln, Sprengmitteln, Schmiermitteln, Farbstoffen, Süßungsmitteln, Aromastoffen, Konservierungsmitteln und Gemischen davon.

8. Zusammensetzung gemäß einem der vorhergehenden Ansprüche, **dadurch gekennzeichnet, dass** die Zusammensetzung als eine Emulsion, Lösung, Dispersion, ein Aerosol, eine Flüssigkeit, ein Gel, Pulver, Granulat, eine Paste oder Tablette formuliert ist; vorzugsweise ist die Zusammensetzung als eine Emulsion, Lösung, ein Aerosol oder eine Tablette formuliert.

9. Träger enthaltend die Verbindung der Formel gemäß einem der Ansprüche 1 oder 2 oder die Zusammensetzung gemäß einem der Ansprüche 3 bis 8.

10. Lockmittelvorrichtung für Insekten der Spezies *Pseudococcus longispinus,* umfassend den Träger gemäß Anspruch 9.

11. Lockmittelvorrichtung gemäß Anspruch 10, **dadurch gekennzeichnet, dass** die effektive durchschnittliche Emission der Verbindung der Formel I, wie in Anspruch 1 oder 2 definiert, oder in der Zusammensetzung gemäß einem der Ansprüche 3 bis 8 oder in dem Träger gemäß Anspruch 9 umfasst, zwischen 0,1 und 250 µg/Tag, vorzugsweise zwischen 0,1 und 100 µg/Tag, stärker bevorzugt zwischen 0,1 und 50 µg/Tag liegt.

12. Verfahren zur Bekämpfung und/oder Überwachung von *Pseudococcus longispinus-*Populationen, umfassend die Verwendung der Verbindung gemäß den Ansprüchen 1 bis 2, die Zusammensetzung gemäß einem der Ansprüche 3 bis 8 oder die Vorrichtung gemäß den Ansprüchen 10 oder 11.

13. Verfahren zur Bekämpfung und/oder Überwachung von *Pseudococcus longispinus-*Populationen gemäß Anspruch 12, wobei die Bekämpfung mittels Anlocken, Fangen, Einfangen durch Paarungsstörung und/oder durch Abtöten von männlichen Individuen der Spezies *Pseudococcus longispinus* erfolgt.

14. Verwendung einer Verbindung gemäß einem der Ansprüche 1 bis 2, einer Zusammensetzung gemäß einem der Ansprüche 3 bis 8, eines Trägers gemäß Anspruch 9 oder einer Vorrichtung gemäß den Ansprüchen 10 oder 11 zur Bekämpfung und/oder Überwachung von *Pseudococcus* longispinus-Populationen.

15. Verfahren zur Synthese einer Verbindung gemäß Anspruch **1,** umfassend die Schritte:
i. Aldol-Kondensation einer Verbindung der Formel **1:** 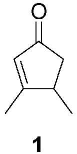 mit p-Formaldehyd, um eine Verbindung der Formel **2** zu erhalten: 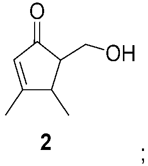
ii. Schützen der Hydroxylgruppe der Verbindung **2** mit einer Hydroxyl-Schutzgruppe PG₁;
iii. Reduktion des Carbonyls des Produkts aus Schritt ii und Schützen der erhaltenen - OH-Gruppe mit einer Hydroxyl-Schutzgruppe PG₂, um eine Verbindung der Formel **3** zu erhalten: 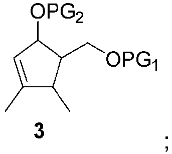
iv. Umlagerung der Verbindung **3** in Verbindung **4:** 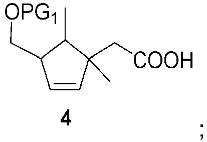
v. Reduktion der Carboxylgruppe der Verbindung **4** und Schützen der erhaltenen Hydroxylgruppe mit einer Acetylgruppe, um Verbindung **5** zu erhalten: 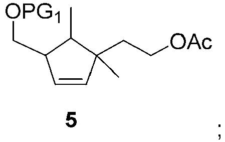
vi. Entschützung der PG1-geschützten Hydroxylgruppe der Verbindung **5** und Jodierung, um Verbindung **6** zu erhalten: 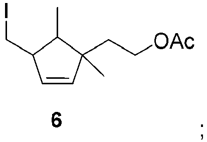
vii. Dehydrohalogenierung der Verbindung 6, um eine Verbindung der Formel I zu erhalten, wobei R gleich Ac ist, oder Dehydrohalogenierung der Verbindung 6 gefolgt von Hydrolyse der Acetylgruppe und Umsetzung mit einem Anhydrid der Formel 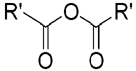 wobei R' ausgewählt ist aus C2-C6-Alkyl oder C3-C6-Cycloalkyl, um eine Verbindung der Formel I zu erhalten, wobei R sich von Ac unterscheidet.

## Revendications

1. Composé de formule I, 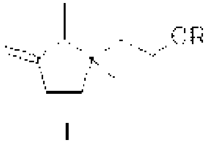 ses stéréoisomères, 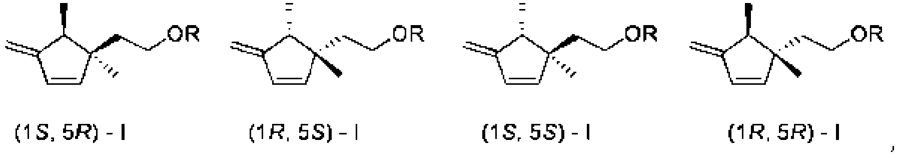 et des mélanges de ceux-ci,
dans lequel R est un groupe -C(=O)R', R' étant choisi parmi un alkyle en C₁ à C₆ ou un cycloalkyle en C₃ à C₆.

2. Composé de formule I selon la revendication 1, dans lequel R est un groupe -C(=O)R', R' étant choisi parmi un méthyle ou un éthyle.

3. Composition pour contrôler et/ou surveiller des populations de *Pseudococcus longispinus* comprenant un composé selon l'une quelconque des revendications 1 à 2.

4. Composition selon la revendication 3, comprenant au moins un ingrédient supplémentaire.

5. Composition selon la revendication 4, dans laquelle l'ingrédient supplémentaire est choisi parmi des substances attractives, des antioxydants, des protecteurs contre le rayonnement, des agents de lutte contre les insectes, des phéromones, des kairomones et des mélanges de ceux-ci.

6. Composition selon l'une quelconque des revendications 3 à 5, comprenant au moins un excipient acceptable du point de vue agricole.

7. Composition selon la revendication 6, dans laquelle l'excipient acceptable du point de vue agricole est choisi parmi des liants, des diluants, des délitants, des lubrifiants, des colorants, des édulcorants, des aromatisants, des conservateurs et des mélanges de ceux-ci.

8. Composition selon l'une quelconque des revendications précédentes, **caractérisée en ce que** la composition est formulée comme une émulsion, une solution, une dispersion, un aérosol, un liquide, un gel, une poudre, un granule, une pâte ou un comprimé, la composition étant de préférence formulée comme une émulsion, une solution, un aérosol ou un comprimé.

9. Support contenant le composé de formule selon l'une quelconque des revendications 1 ou 2 ou la composition selon l'une quelconque des revendications 3 à 8.

10. Dispositif attractif pour insectes de l'espèce *Pseudococcus longispinus* comprenant le support selon la revendication 9.

11. Dispositif attractif selon la revendication 10, **caractérisé en ce que** l'émission moyenne efficace du composé de formule I, tel que défini dans la revendication 1 ou 2, ou compris dans la composition selon l'une quelconque des revendications 3 à 8 ou dans le support selon la revendication 9, est comprise entre 0,1 et 250 µg/jour, de manière préférée entre 0,1 et 100 µg/jour, de manière plus préférée entre 0,1 et 50 µg/jour.

12. Procédé de contrôle et/ou de surveillance de populations de *Pseudococcus longispinus* comprenant l'utilisation du composé selon les revendications 1 à 2, de la composition selon l'une quelconque des revendications 3 à 8 ou du dispositif selon les revendications 10 ou 11.

13. Procédé de contrôle et/ou de surveillance de populations de *Pseudococcus longispinus* selon la revendication 12, dans lequel le contrôle est effectué en attirant, attrapant, capturant par confusion sexuelle et/ou par mort des individus mâles appartenant à l'espèce *Pseudococcus longispinus.*

14. Utilisation d'un composé selon l'une quelconque des revendications 1 à 2, d'une composition selon l'une quelconque des revendications 3 à 8, d'un support selon la revendication 9 ou d'un dispositif selon les revendications 10 ou 11 pour le contrôle et/ou la surveillance de populations de *Pseudococcus longispinus.*

15. Procédé pour la synthèse d'un composé selon la revendication 1, comprenant les étapes suivantes :
i. condensation aldolique d'un composé de formule 1 : 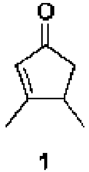 avec du p-formaldéhyde pour obtenir un composé de formule 2 : 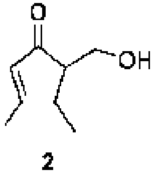
ii. protection du groupe hydroxyle du composé 2 avec un groupe protecteur d'hydroxyle PG₁,
iii. réduction du carbonyle du produit de l'étape ii et protection du groupe -OH résultant avec un groupe protecteur d'hydroxyle PG₂ pour obtenir un composé de formule 3 : 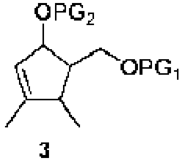
iv. réarrangement du composé 3 en composé 4 : 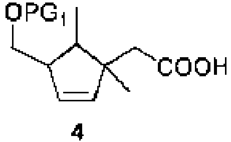
v. réduction du groupe carboxyle du composé 4 et protection du groupe hydroxyle résultant avec un groupe acétyle pour obtenir le composé 5 : 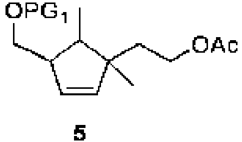
vi. déprotection du groupe hydroxyle protégé par PG₁ du composé 5 et iodation pour obtenir le composé 6 : 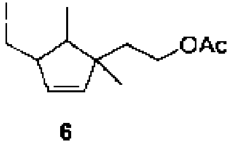
vii. déshydrohalogénation du composé 6 pour obtenir un composé de formule 1, dans laquelle R est Ac, ou déshydrohalogénation du composé 6 suivie d'une hydrolyse du groupe acétyle et d'une réaction avec un anhydride de formule 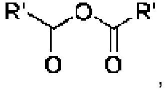 dans laquelle R' est choisi parmi un alkyle en C₂ à C₆ ou un cycloalkyle en C₃ à C₆, pour obtenir un composé de formule I, dans laquelle R est différent d'Ac.
